# EUROPEAN PATENT SPECIFICATION

(11) **EP 1 600 207 B1**
(45) Date of publication and mention of the grant of the patent: **30.04.2014**
(21) Application number: 04716708.5
(22) Date of filing: 03.03.2004
(51) Int. Cl.: B01F 17/56, A23L 1/035, A23L 1/22, A23L 1/24

(54) **EMULSIFIER, PROCESS FOR PRODUCING THE SAME AND EMULSIFIED COMPOSITION USING THE EMULSIFIER**
EMULGATOR, VERFAHREN ZUR HERSTELLUNG DESSELBEN UND DEN EMULGATOR VERWENDENDE EMULGIERTE ZUSAMMENSETZUNG
EMULSIFIANT, PROCEDE DE PRODUCTION DUDIT EMULSIFIANT ET COMPOSITION EMULSIFIEE UTILISANT LEDIT EMULSIFIANT

(30) Priority: 04.03.2003 JP 2003057839
(43) Date of publication of application: 30.11.2005
(73) Proprietor: Fuji Oil Company, Ltd., Osaka-shi, Osaka 542-0086 (JP)
(72) Inventor: NAKAMURA, Akihiro, Ibaraki 300-2436 (JP); TAKAHASHI, Taro, Ibaraki 300-2436 (JP); TOBE, Junko, Ibaraki 3002436 (JP)
(74) Representative: Boult Wade Tennant
(86) International application number: PCT/JP2004/002670
(87) International publication number: WO 2004/078335

(56) References cited:
- WO-A1-02/26788
- JP-A- 3 058 758
- JP-A- 7 031 863
- JP-A- 11 346 668
- FURUTA H ET AL: "Extraction of Water-soluble Soybean Polysaccharides under Acidic Conditions" BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN LNKD- DOI:10.1271/BBB.62.2300, vol. 62, no. 12, 1 January 1998 (1998-01-01), pages 2300-2305, XP002383358 ISSN: 0916-8451
- YOSHII H ET AL: "HYDROLYSIS KINETICS OF OKARA AND CHARACTERIZATION OF ITS WATER-SOLUBLE POLYSACCHARIDES" POSTHARVEST BIOLOGY AND TECHNOLOGY, ELSEVIER, NL, vol. 60, no. 9, 1 January 1996 (1996-01-01), pages 1406-1409, XP009080733 ISSN: 0925-5214
- MAEDA H: "Soluble soybean polysaccharide" 1 January 2000 (2000-01-01), HANDBOOK OF HYDROCOLLOIDS, WOODHEAD PUBLISHING, GB LNKD- DOI:10.1016/B978-044450178-3/50003-2, PAGE(S) 309 - 320 , XP008098734 ISBN: 978-0-8493-0850-5 * paragraphs [17.2], [ 17.5] *
- H. SHIMIZU: FOODS FOOD INGREDIENTS J. JAPAN, no. 201, 2002, pages 1-1, XP002592378

## Description

### Technical Field

The present invention relates to an emulsifier which is excellent in emulsifying capability including emulsification ability and emulsion stabilisation ability in particular, to a high-molecular type emulsifier of soybean origin having an extremely good emulsifying capability, a process for producing the same, and to an emulsion using the emulsifier.

### Background Art

Generally, emulsifiers can be roughly classified into monomolecular emulsifiers and high-molecular emulsifiers. The monomolecular emulsifiers, which are so-called surfactants, include fatty acid soaps, glycerin esters, sugar esters, and the like. High-molecular emulsifiers include natural substances such as gum arabic and casein, as well as synthetic products such as polyacrylic acid salts and polyvinyl alcohols.

These emulsifiers are used alone or in an appropriate combination thereof depending upon applications. Monomolecular emulsifiers generally have strong emulsification ability and can provide emulsions even when using a small amount thereof. In some cases, however, monomolecular emulsifiers have problems of emulsion stabilization ability, that is, there remain such problems that they are influenced by change in pH, or that their emulsification properties are lost due to change in concentration caused by addition of a salt or dilution

On the other hand, regarding high-molecular emulsifiers, for example, in case of an emulsifier such as gum arabic, which is a natural high-molecule substance, there are difficulties in its practical use because it is required to use it at high concentration relative to a fat ingredient in an emulsion in order to form a stable emulsion state, and its market price varies because of its unstable harvest. While xanthan gum or the like is used as an emulsifier for dressings, xanthan gum renders the dressings highly viscous, and such dressings do not always have satisfactory feeling for eating. Further, while casein or the like used in creams such as coffee cream, such cream is not always satisfactory because of its high susceptibility to pH changes and the breakdown of its emulsions upon dilution. Regarding substances other than natural products, synthetic products such as polyacrylic acid salts and polyvinyl alcohols can be used, but they are often limited in their applications because of their insufficient emulsification properties. Under these circumstances, there is a demand for natural high-molecular circumstances, there is a demand for natural high-molecular emulsifiers which have high emulsifying capabilities, which are suited for wide variety of applications, and which can be supplied stably as commercial products.

For solving these problems, the present applicant has filed a patent application directed to an emulsifier comprising as an effective ingredient a water-soluble polysaccharide of soybean origin (Patent Document 1). The water-soluble soybean polysaccharide extracted at a high temperature at pH 4.5, which is a slightly acidic pH in the vicinity of the isoelectric point of soybean protein is observed to have emulsifying ability. This, however, is not always a satisfactory function in systems having a higher fat ingredient content.

As for the production of water-soluble polysaccharides of soybean origin, generally, processes which comprise extracting them from raw materials containing the water-soluble polysaccharide fractions with water or hot water in an alkaline, neutral or acidic region; or which comprise hydrolyzing the raw materials have been known. For example, proposed is a process for producing a water-soluble soybean polysaccharide which exhibits fluidity in a wide temperature range from a low temperature to a high temperature and also exhibits an extremely low viscosity, the process comprising extracting soybeans as a raw material within a slightly acidic region of pH 3 to 7 (Patent Document 2).

As applications of water-soluble soybean polysaccharides extracted at a slightly acidic pH in the vicinity of the isoelectric point, namely, pH 4.5, various proposals have been made, for example, dispersion stabilizers for acidic milk beverages (Patent Document 3), loosening improvers for noodles and processed cereal foods (Patent Document 4), film coating agents (Patent Document 5), and emulsifiers (Patent Document 1: JP 6-121922 A) are proposed.

Furuta H et al: "Extraction of water-soluble soybean polysaccharides under acidic conditions" Bioscience Biotechnology Biochemistry, Japan Society for Bioscience, Biotechnology, and Agrochemistry, Tokyo, Japan LNKD-DOI:10.1271/BBB.62.2300, vol. 62, no. 12, 1 January 1998 (1998-01-01), pages 2300-2305 is directed at the extraction of water-soluble soybean polysaccharides under acidic conditions. In particular, Furuta H et al discloses the extraction of water-soluble soybean polysaccharide (SSPS) and that the extraction ratio of SSPS is highest at temperatures from 100 to 120°C at a pH value of 2. Furthermore, Furuta H *et al* suggests that SSPS could be used as a gelling agent, a thickening agent and/or a stabilizing agent during food processing.

Yoshii H et al: "Hydrolysis kinetics of okara and characterization of its water-soluble polysaccharides" Postharvest Biology and Technology, Elsevier, NL, vol. 60, no. 9, 1 January 1996 (1996-01-01), pages 1406-1409 is directed at the hydrolysis kinetics of okara and characterization of its water-soluble polysaccharides. In particular, Yoshii H *et al* discloses the extraction and/or hydrolysis of water-soluble polysaccharides (WSP) with or without a chelating agent at a pH of 4.5 which is the isoelectric point of soybean protein and the use of the obtained WSP as an emulsifier.

Maeda H: "Soluble soybean polysaccharide" 1 January 2000 (2000-01-01), Handbook of Hydrocolloids, Woodhead Publishing, GB LNKD- DOI:10.1016/B978-044450178-3/50003-2, pages 309-320 is directed at a soluble soybean polysaccharide. In particular, Maeda H discloses extracting the water soluble soybean polysaccharide (SSPS) in weak acid conditions and that SSPS can be used *inter alia* for emulsifying and/or emulsion stabilising. Maeda H discloses the emulsification of an amount of fat ingredient up to 2 times larger than the soybean polysaccharide. H. Shimizu: Foods Food Ingredients J. Japan, no. 201, 2002, page 1 is directed at the manufacture of water-soluble soybean polysaccharide from soybean food by-product (okara). In particular, H. Shimizu discloses the extraction of water-soluble soybean polysaccharide (Soybean polysaccharide) under weak acidic conditions and that the polysaccharide can be used *inter alia* in food applications such as a dietary raw fibre, as a stabilizer and as a non-stick agent. H. Shimizu also describes that the extraction at a pH value close to the isoelectric point of soybean protein is desirable.

### (List of Prior Art)

Patent Document 1: JP 6-121922 A
Patent Document 2: JP 3-236759 A
Patent Document 3: JP 5-7449 A
Patent Document 4: JP 6-121647 A
Patent Document 5: JP-A 9-70285

By the way, emulsifiers used for various applications are required to have strong emulsification ability sufficient to exert functions even when used in a small amount and have a high emulsion stability sufficient to maintain a stable state for a long period of time even when in the form of emulsion products. Further, they are required to cause no incompatible mouth feel due to thickening or the like, when used for foods.

For example, monomolecular emulsifiers such as sugar esters generally have strong emulsification ability, but they tend to be affected by pH change. They, therefore, still have emulsion stability problems, that is, the emulsification ability is lost due to concentration change caused by addition of a salt or dilution. On the other hand, regarding high-molecular emulsifiers, gum arabic is widely used as an emulsifier for emulsified flavors or powdery flavors, xanthan gum is used for dressings, and casein is used for cream such as coffee cream. They, however, have various problems such as those mentioned above. Further, a water-soluble soybean polysaccharide extracted at a slightly acidic pH in the vicinity of the isoelectric point at a high temperature provides an emulsion with high stability, but it exerts only a weak emulsification ability by itself. It can exert a sufficient emulsifying capability only in the case where the fat ingredient/emulsifier weight ratio is 4 times or less and its application field is limited. Accordingly, the current situation is not necessarily satisfactory. The wording "stable emulsion" used herein refers to an emulsion "which has an emulsion particle diameter of 5 µm or less and in which an emulsion state is maintained with stability even when the emulsion is stored at 5°C for one month".

### Disclosure of the Invention

The present invention provides an emulsifier with a high emulsifying capability which can produce a stable emulsion by utilizing a combination of its emulsification ability and its emulsion stabilization ability, and particularly provides an emulsifier which is a natural high-molecule type emulsifier, but which exerts a sufficient emulsifying effect to emulsions having a higher fat ingredient content relative to the emulsifier.

The present inventors have studied to solve the aforementioned problems. As a result, they have found that in the extraction of a water-soluble polysaccharide from soybeans or a processed soybean product, the emulsifying capability of a resulting polysaccharide varies greatly depending upon the extraction pH, and that a remarkably improved emulsifying capability is exerted by an extract which contains, as the active ingredient, a water-soluble polysaccharide obtained by heat extraction conducted at a high temperature not at a pH in the vicinity of the isoelectric point of the protein which is suitable for separating the protein and the polysaccharide, but within a range where the protein dissolves to some extent, that is, a pH region lower than the isoelectric point. Thus, they have completed the present invention.

That is, the present invention provides an emulsifier which comprises, as an active ingredient, a water-soluble soybean polysaccharide extracted from soybeans or a processed soybean product at a pH of 2.4 to 4.0 with heating to a temperature of 100°C or higher, wherein the emulsifier has an emulsifying capability sufficient to form a stable emulsion of a composition containing a fat ingredient in an amount 5 times larger than the emulsifier or more in terms of the weight ratio; a production process for obtaining the emulsifier; and an emulsified composition using the emulsifier. The upper limit of the ratio of the fat ingredient is not specifically limited and a ratio of about 100 times will cause no problems.

### Best Mode for Carrying Out the Invention

Preferred embodiments of the present invention will be illustrated in detail below. According to the present invention, an emulsifier comprising a water-soluble soybean polysaccharide as an active ingredient which exhibits a high emulsification activity even at an fat ingredient/emulsifier weight ratio of 5 or more is obtained by extracting soybeans or a processed soybean product with heating to a temperature exceeding 100°C at a pH lower than the isoelectric point of soybean protein, preferably pH 2.4 to 4.0, more preferably within an acidic pH of pH 2.8 to 3.7, and most preferably within an acidic pH of pH 3.0 to 3.5. In addition, an emulsified composition using the emulsifier is provided.

The soybean raw material to be used in the present invention is preferably cotyledons of soybeans. More preferred is the so-called "okara (soybean curd refuse)", which is by-produced during the preparation of tofu or soybean protein isolate, because it is rich in polysaccharides. In case of using "okara" of tofu production origin as a raw material, water-soluble low molecular weight fractions have been removed in advance. Further, in case of using "okara" by-produced during the preparation of soybean protein isolate, oil-soluble components have also been removed in advance. Therefore, it is advantageous to use them as a raw material.

In the present invention, heat extraction from these raw materials is carried out at first. The pH at the time of the heat extraction is adjusted to a pH lower than the isoelectric point of soybean protein, preferably to a pH of 2.4 to 4.0, and more preferably to a pH of 3.0 to 3.5. While the acid to be used in this operation is not particularly limited, when the emulsifier obtained is intended to be used in food applications, the acid should be that permitted to be used in the food industry such as hydrochloric acid, phosphoric acid, sulfuric acid, lactic acid, citric acid and oxalic acid.

It is important that the heat extraction temperature is at 100 °C or higher. A heat extraction temperature lower than 100°C is undesirable from a practicality aspect because a long time is required for extraction or the yield decreases even if the pH is adjusted to the above-mentioned ranges. In addition, it is impossible to obtain water-soluble soybean polysaccharides with sufficient emulsification activities in some cases. There is no particular upper limit with the heating temperature, but to conduct the extraction at an extremely high temperature is undesirable because it tends to cause side reactions or coloration. It is recommended to conduct it at a temperature typically of 180°C or lower, and preferably 150°C or lower.

By conducting extraction within the aforementioned pH ranges, the emulsifying capability of the water-soluble soybean polysaccharides is drastically improved. The reason why the emulsifying capability is improved is not clear in detail. It, however, is considered that change in conformation of polysaccharides caused by pH difference, sugar/protein interaction and sugar-protein balance, i.e., hydrophilic-hydrophobic balance in the water-soluble soybean polysaccharide to be extracted fall within ranges suitable for emulsification. Particularly with respect to the relationship with protein, it has been reported that, in the extraction of polysaccharides, extraction in the vicinity of the isoelectric point of protein, which is the conditions wherein the protein is most slightly soluble, is desirable for avoiding the influence of the protein. It, however, is considered that in the improvement in emulsifier functions, an effect that the protein is rendered soluble to some extent at a pH lower than the isoelectric point is produced, thereby amplifying the polysaccharide-protein interaction.

After the heat extraction, a solid matter and an extract are separated by a conventional method such as filtration or centrifugation. In some cases, to neutralize the reaction mixture after heating to a neutral or slightly acidic region beyond the isoelectric point is advantageous for preservative property of the emulsifier or its application for food products. Subsequently, the extract is subjected, if necessary, to neutralization, desalting, and purification treatment for removing hydrophobic substances or low molecular weight substances. Examples of the purification method include activated carbon treatment, or resin adsorption treatment, or reprecipitation using a polar solvent such as methanol, ethanol, isopropanol or acetone, ultrafiltration, reverse osmosis, gel filtration, dialysis, an ion exchange resin method, electrodialysis, or an ion exchange membrane method. These methods can be used alone or in combination of two or more thereof. In particular, when using reprecipitation using a polar solvent, ultrafiltration, reverse osmosis, gel filtration, or dialysis, it is possible to remove also a variety of low molecular weight substances. In Case of desalting purification, desirably, desalting is conducted so that the ash content in the polysaccharide after the treatment becomes 15% by weight or less, preferably 5 to 10% by weight.

Although the water-soluble soybean polysaccharide thus obtained can be used regardless of its molecular weight, a preferred average molecular weight is from several thousands to several millions, and a specifically preferred average molecular weight is from 5,000 to 1,000,000, more preferably 10,000 to 300,000. If the molecular weight is too large, the viscosity will increase to cause difficulty in emulsification. The average molecular weight of the water-soluble soybean polysaccharide used herein is that determined by gel filtration HPLC using a TSK-GEL G-5000PWXL column using a standard pullulan (available from Showa Denko K.K.) as a standard substance.

The solution viscosity, which is a measure of the molecular weight of the water-soluble soybean polysaccharide solution obtained in the present invention, is preferably, for example, 150 mPa·s or less, more preferably 50 mPa·s or less, and even more preferably 30 mPa·s or less, in the case of a 10% aqueous solution.

At the time of conducting the aforementioned purification treatment, demethoxylation treatment can be conducted by a known method (JP 5-262802 A), for example, by heat-treating within an alkaline region before or after the purification treatment.

Herein, the sugar content in the water-soluble soybean polysaccharide produced was measured by the phenol sulfuric acid method. The uronic acid content was measured by the Blumenkrantz method. The crude protein content was measured by the semi-micro Kjeldahl method, followed by multiplying the amount of nitrogen determined by a nitrogen coefficient, 6.25.

Although gum arabic is currently widely used for emulsified flavors, there is anxiety with its supply. In light of this, modified starch or the like have been developed. These, however, cause great change in conditions with time and, therefore, are not satisfactory from stability aspect.

When the water-soluble soybean polysaccharide in the present invention is employed as an emulsifier for an emulsified flavor, it is possible to provide a state of emulsion superior to that provided by using gum arabic or modified starch in stabilities such as heat resistance, acid resistance, salt resistance and alcohol resistance stability as well as suspension stability.

When it is used in dressings, the resultant dressings have a lower viscosity and light mouth feel as compared with those provided by using xanthan gum or starch. In case of using it for cream such as coffee whiteners, it is possible to obtain cream which hardly causes emulsion breakdown against change in pH or dilution.

The water-soluble soybean polysaccharide in the present invention can also be applied widely applications other than foods. For example, it can also be applied to cosmetics and pharmaceutical cream, such as hand cream and ointments. It can also be used for agricultural chemicals such as insecticides and herbicides of oil-in-water emulsion type. When the water-soluble soybean polysaccharide is used as an emulsifier, a state of emulsion stable for a long period of time is obtained. In addition, the emulsion is stable after being sprayed and is superior in durability of the effect.

In the present invention, while the water-soluble soybean polysaccharide can be used alone as an emulsifier, it can produce a synergistic effect when being used together with an existing emulsifier.

Examples of existing monomolecular emulsifiers include various anionic surfactants, typically, fatty acid soaps, cationic surfactants such as quaternary ammonium salts, nonionic surfactants such as glycerin fatty acid esters and sugar esters, and amphoteric surfactants such as lecithin.

Examples of existing high molecular emulsifiers include natural emulsifiers such as agar, carrageenan, furcellaran, tamarind seed polysaccharides, angelica gum, gum karaya, pectin, xanthan gum, sodium alginate, tragacanth gum, guar gum, locust bean gum, pullulan, jellan gum, gum arabic, gelatin, whey and other albumins, casein sodium and various starches. Examples of semi-synthetic sizing agents include carboxymethyl cellulose (CMC), methyl cellulose (MC), hydroxyethyl cellulose (HEC), propylene glycol alginate and modified starches typified by soluble starches. Examples of synthetic sizing agents include polyvinyl alcohols and sodium polyacrylate.

In some cases, the emulsifier of the present invention exhibits an improved effect when being used together with one or more kinds of aforementioned emulsifiers, and can make up for drawbacks of these emulsifiers.

In some cases, the stability of the emulsion can be improved by addition of saccharides including sucrose and starch syrup, polyhydric alcohols such as glycerin, D-sorbitol and propylene glycol and acidulants such as lactic acid, vinegar, citric acid and malic acid to the water phase. Further, other additives, for example, anti-phase. Further, other additives, for example, anti-discoloring agents such as L-ascorbic acid and its derivatives or aminocarbonyl reaction products, and preservatives may also be included.

The oil phase used for the emulsified composition of the present invention may be any oily substances which are slightly soluble in water, and examples thereof include common fats and oils, oil-soluble aromatics, oil-soluble pigments, waxes, insecticides, herbicides, oil-soluble pharmaceuticals and oil-soluble reagents. The present invention, therefore, can be widely and generally used in industrial products necessary for living, typical examples of which include emulsified flavors, foods such as mayonnaise, dressings and cream, cosmetics such as hand cream, medicines such as ointment and agricultural chemicals such as insecticides.

The present invention will be illustrated in more detail with reference to the following Examples and Comparative Examples, which are only exemplary and, therefore, the spirit and scope of the present invention are not intended to be limited thereto. In the Examples, all the parts and percentages are by weight.

### Examples

### Example 1

To raw okara obtained during a production step of soybean protein isolate was added twice amount of water. The mixture was adjusted to pH 2.5 with hydrochloric acid and heat-extracted at 120°C for 1.5 hr. After cooling, the heat-extracted slurry had a pH of 2.48. The slurry recovered was adjusted to pH 5.0, and then centrifuged (10000 G × 30 min) to separate it into a supernatant and a precipitate. The precipitate thus separated was further washed with equal amount water, and centrifuged. The supernatant thus formed was combined with that obtained previously, and the combined supernatant was subjected to desalting treatment by electrodialysis, and then dried to obtain the water-soluble soybean polysaccharide (A).

### Example 2

To raw okara obtained during a production step of soybean protein isolate was added twice amount of water. The mixture was adjusted to pH 3.0 with hydrochloric acid and heat-extracted at 120°C for 1.5 hr. After cooling, the heat-extracted slurry had a pH of 2.98. The slurry recovered was adjusted to pH 5.0, and then centrifuged (10000 G × 30 min) to separate it into a supernatant and a precipitate. The precipitate thus separated was further washed with equal amount of water, and centrifuged. The supernatant thus formed was combined with that obtained previously, and the combined supernatant was subjected to desalting treatment by electrodialysis, and then dried to obtain the water-soluble soybean polysaccharide (B).

### Example 3

To raw okara obtained during a production step of soybean protein isolate was added twice amount of water. The mixture was adjusted to pH 3.5 with hydrochloric acid and heat-extracted at 120°C for 1.5 hr. After cooling, the heat-extracted slurry had a pH of 3.57. The slurry recovered was adjusted to pH 5.0, and then centrifuged (10000 G × 30 min) to separate it into a supernatant and a precipitate. The precipitate thus separated was further washed with equal amount of water, and centrifuged. The supernatant thus formed was combined with that obtained previously, and the combined supernatant was subjected to desalting treatment by electrodialysis, and then dried to obtain the water-soluble soybean polysaccharide (C).

### Example 4

To raw okara obtained during a production step of soybean protein isolate added twice amount of water. The mixture was adjusted to pH 4.0 with hydrochloric acid and heat-extracted at 120°C for 1.5 hr. After cooling, the heat-extracted slurry had a pH of 4.00. The slurry recovered was adjusted to pH 5.0, and then centrifuged (10000 G × 30 min) to separate it into a supernatant and a precipitate. The precipitate thus separated was further washed with equal amount of water, and centrifuged. The supernatant thus formed was combined with that obtained previously, and the combined supernatant was subjected to desalting treatment by electrodialysis, and then dried to obtain the water-soluble soybean polysaccharide (D).

### Comparative Example 1

To raw okara obtained during a production step of soybean protein isolate was added twice amount of water. The mixture was adjusted to pH 4.5 with hydrochloric acid and heat-extracted at 120°C for 1.5 hr. After cooling, the heat-extracted slurry had a pH of 4.61. The slurry recovered was adjusted to pH 5.0, and then centrifuged (10000 G × 30 min) to separate it into a supernatant and a precipitate. The precipitate thus separated was further washed with equal amount of water, and centrifuged. The supernatant thus formed was combined with that obtained previously, and the combined supernatant was subjected to desalting treatment by electrodialysis, and then dried to obtain a water-soluble soybean polysaccharide.

### Comparative Example 2

To raw okara obtained during a production step of soybean protein isolate was added twice amount of water. The mixture was adjusted to pH 2.0 with hydrochloric acid and heat-extracted at 80°C for 3.0 hr. After cooling, the heat-extracted slurry had a pH of 2.10. The slurry recovered was adjusted to pH 5.0, and then centrifuged (10000 G × 30 min) to separate it into a supernatant and a precipitate. The precipitate thus separated was further washed with equal amount of water, and centrifuged. The supernatant thus formed was combined with that obtained previously, and the combined supernatant was subjected to desalting treatment by electrodialysis, and then dried to obtain a water-soluble soybean polysaccharide.

The results of the analysis of the water-soluble soybean polysaccharides obtained in the methods described above are summarized in the following Table 1.

**(Table 1)**

| | Example 1 | Example 2 | Example 3 | Example 4 | Comparative Example 1 | Comparative Example 2 |
|---|---|---|---|---|---|---|
| pH before heating | 2.50 | 3.00 | 3.50 | 4.00 | 4.50 | 2.00 |
| Heating conditions | 120°C | ← | ← | ← | ← | 80°C |
| | 1.5 hr | ← | ← | ← | ← | 3.0 hr |
| pH after heating | 2.48 | 2.98 | 3.57 | 4.00 | 4.61 | 2.10 |

| Composition ratio(% by weight on the basis of dried product) | | | | | | |
|---|---|---|---|---|---|---|
| Crude protein | 20.4 | 17.4 | 14.7 | 12.8 | 11.1 | 14.9 |
| Crude ash | 4.2 | 4.1 | 3.9 | 4.1 | 4.2 | 4.7 |
| All sugar | 78.4 | 81.5 | 85.1 | 82 | 86.1 | 73.2 |
| Uronic acid | 13.4 | 13.7 | 13.3 | 13.3 | 14 | 10.8 |
| Average molecular weight | 44000 | 68000 | 105000 | 257000 | 385000 | - |
| 10% Solution viscosity (mPa·s) | 6.5 | 9.0 | 17.0 | 37.0 | 51.0 | - |

### Test Example 1

The emulsifying capabilities of the products of the present invention obtained in Examples 1 to 4 and the water-soluble soybean polysaccharides obtained in Comparative Examples 1 and 2 were examined. In addition, as Comparative Example 3, the emulsifying capability of a commercially available sucrose fatty acid ester "DK ESTER SS" (Dai-ichi Kogyo Seiyaku Co., Ltd.) was also examined.

Method: A test solution having the formulation shown below was subjected to emulsification treatment for 30 seconds twice by using an ultrasonic emulsifier (manufactured by Kaijo Denki Co., Ltd.; 5281 type piezoelectric transducer) and then to measurement of a median particle diameter using a laser diffraction particle size analyzer "SALD-2000A" (manufactured by Shimadzu Corp.). The results are shown in the following Table 2. The buffer solutions were a 100 mM sodium citrate-HCl buffer solution (pH 4.0) and 100 mM sodium phosphate-HCl buffer solution (pH 7.0)

**(Table 2)**

| Oil/emulsifier ratio | 26.7 times | 6.7 times | 1 time |
|---|---|---|---|
| Oil | Soybean oil 40.0% | Soybean oil 25.0% | Lemon oil 9.0% |
| Water-soluble soybean polysaccharide | 1.5% | 3.8% | 9.0% |
| Water | 28.5% | 33.7% | - |
| Buffer solution of pH 4.0 or 7.0 | 30.0% | 37.5% | 64.5% |
| Glycerol | - | - | 17.5% |

Regarding the median particle diameter of each emulsion obtained by the method described above, the measurement result obtained immediately after the emulsification is shown in Table 3 and that obtained after one-month preservation at 5°C is shown in Table 4.

**(Table 3)**

| | | Example 1 | Example 2 | Example 3 | Example 4 | Comparative Example 1 | Comparative Example 2 | Comparative Example 3 |
|---|---|---|---|---|---|---|---|---|
| Oil/ emulsifier ratio | Buffer solution pH | (µm) | (µm) | (µm) | (µm) | (µm) | (µm) | (µm) |
| 26.7 times | 4.0 | 7.88 | 2.37 | 3.11 | 13.6 | 19.9 | 7.56 | 1.77 |
| 26.7 times | 7.0 | 3.81 | 2.44 | 3.98 | 15.4 | 21.5 | 7.44 | 1.57 |
| 6.7 times | 4.0 | 2.05 | 1.35 | 1.29 | 2.92 | 6.56 | 3.38 | 1.42 |
| 6.7 times | 7.0 | 2.48 | 1.41 | 1.36 | 3.00 | 5.83 | 3.14 | 1.23 |
| 1.0 time | 4.0 | 1.00 | 0.679 | 0.536 | 0.592 | 0.771 | 2.75 | 0.817 |

**(Table 4)**

| | | Example 1 | Example 2 | Example 3 | Example 4 | Comparative Example 1 | Comparative Example 2 | Comparative Example 3 |
|---|---|---|---|---|---|---|---|---|
| Oil/ emulsifier ratio | Buffer solution pH | (µm) | (µm) | (µm) | (µm) | (µm) | (µm) | (µm) |
| 26.7 times | 4.0 | 8.75 | 2.52 | 3.33 | 14.5 | 25.3 | 8.46 | 5.63 |
| 26.7 times | 7.0 | 4.16 | 2.57 | 4.36 | 16.5 | 24.2 | 8.18 | 1.80 |
| 6.7 times | 4.0 | 2.13 | 1.39 | 1.33 | 3.14 | 7.82 | 3.47 | 3.11 |
| 6.7 times | 7.0 | 2.54 | 1.45 | 1.39 | 3.14 | 5.90 | 3.21 | 1.40 |
| 1.0 time | 4.0 | 1.07 | 0.706 | 0.549 | 0.629 | 0.825 - | 2.77 | Agglomeration |

As shown above, the emulsification activity in a high fat ingredient system was remarkably improved in the products of the present invention, i.e., the products prepared in Examples 1 to 4 as compared with water-soluble soybean polysaccharides obtained in Comparative Examples 1 and 2. In addition, as compared with the commercially available sucrose fatty acid ester of Comparative Example 3, the storage stability at pH 4.0 was remarkably improved. In the products of the present invention, i.e., the products prepared in Examples 1 and 4, since the median particle diameters in the systems were large such as a ratio of an fat ingredient/emulsifier of 26.7 times, the range of heat extraction pH, where a good emulsification activity was achieved, was from 2.4 to 4.0, preferably pH of from 3.0 to 3.5.

On the other hand, as shown by the results of Comparative Example 2, no sufficient emulsifying capability was obtained at a heat-extraction temperature lower than 100°C even though the heat extraction pH was adjusted to 3.0. In order to obtain a good emulsifying activity, heating at a temperature of 100°C or higher was required.

### Example 5

One part of the product of the present invention prepared in Example 2 was suspended in 12 parts of purified coconut oil and was stirred and mixed with a homomixer. In addition, 24 parts of water, 15 parts of granulated sugar, 4.5 parts of glucose and 0.5 parts of dextrin were added thereto and the mixture was heated to 60°C to conduct preliminary emulsification. Subsequently, the mixture was emulsified at 100 kgf/cm² by using a high pressure homogenizer. Then, 30 parts of fruit puree and 5 parts by weight of lemon juice were added thereto to obtain a soft ice cream mix of pH 3.15. The soft ice cream mix favorably had an emulsion particle diameter of 1.8 µm though the amount of the fat ingredient used was 12 times as much as that of the water-soluble soybean polysaccharide. Furthermore, when acidic soft ice cream was prepared by freezing the soft ice cream mix with whipping it by means of an ice cream maker, the acidic soft ice cream had very good taste with both smooth mouth feel and body while having a noticeable taste of fruits.

### Example 6

In 13.5 parts of water, 1.5 parts of the product of the present invention prepared in Example 2, 2.5 parts of table salt and 0.5 part of sodium glutamate were dissolved. Then, 12.5 parts of vinegar was further added to the resulting solution, and the solution was placed in a mixer. Into the mixer, 70 parts of purified canola oil was added slowly to conduct emulsification with the solution, thereby preparing a mayonnaise-like dressing. The mayonnaise-like dressing had good taste and properties with an emulsion particle diameter as small as 3.2 µm though the amount of the fat ingredient used was 47 times as much as that of the water-soluble soybean polysaccharide. Furthermore, it exhibited no change in conditions and was stable even after a three-month cold stowage.

### Comparative Example 4

A mayonnaise-like dressing was prepared according to the same manner as Example 6 except for using the comparative example product prepared in Comparative Example 1. In the mayonnaise-like dressing, the fat ingredient was separated and, therefore, the dressing was of no commercial value.

### Example 7

A mixed oil composed of 13 parts of SAIB (sucrose acetate isobutyrate) and 7 parts of an orange oil was dispersed in an aqueous phase, which was prepared by dissolving 4.5 parts of the product of the present invention prepared in Example 2 in 40.5 parts of water and further adding 35 parts of glycerol, to make up the volume of the dispersion to 100 parts. The dispersion was adjusted to pH 4.0 with a 50% citric acid solution and then emulsified with a homogenizer (100 kgf/cm²). The emulsified flavor had an emulsion particle diameter as small as 0.6 µm though the amount of the fat ingredient used was 4.4 times as much as that of the water-soluble soybean polysaccharide, and it was stable with exhibiting no changes in particle diameter and conditions even after storage in a refrigerator for three months. Then, 120 parts of granulated sugar and 2 parts of citric acid were dissolved in 880 parts of water, and one part of the above-described emulsified flavor was added thereto to obtain an orange-like soft drink. The drink was quite stable even after the elapse of 3 months.

### Example 8

In an aqueous phase prepared by dissolving 4 parts of the product of the present invention product prepared in Example 2 in 60 parts of water, 20 parts of lemon oil was dispersed to make up the volume of the dispersion to 100 parts. The dispersion was adjusted to pH 4.0 with a 50% citric acid solution and then emulsified with a homogenizer (100 kgf/cm²) and then spray dried. The powdery flavor had an emulsion particle diameter as small as 1.2 µm though the amount of the fat ingredient used was 5 times as much as that of the water-soluble soybean polysaccharide. Moreover, the vaporization of the flavor component by the spray dry treatment was controlled.

### Example 9

In 69 parts of water, 4 parts of the product of the present invention prepared in Example 2 and 2 parts of sodium caseinate were dissolved. To the solution was added 25 parts of purified coconut oil containing 0.1 parts of commercially available milk flavor "Milk FT-013" (manufactured by Takasago International Corp.) at 70°C, and the mixture was pre-emulsified with a homomixer. Then, the resultant was emulsified (300 kgf/cm²) with a high-pressure homogenizer to obtain a whitener for coffee. The whitener had an emulsion particle diameter as small as 0.8 µm though the amount of the fat ingredient used was used 6.3 times as much as that of the water-soluble soybean polysaccharide and it was stable even after storage in a refrigerator for one month after sterilizing treatment. The whitener was added to coffee (adjusted to pH 6.8 with sodium hydrogen carbonate) containing 8% of sugar, and the coffee was then sterilized at 121°C for 30 min and stored at 60°C for 3 months. As a result, it remained stable in a suspension form.

### Example 10

In 60 parts of water, 3 parts of the product of the present invention prepared in Example 2 was dissolved, and 10 parts of propylene glycol, 0.5 parts of triethanolamine, 0.5 parts of an oily flavor, and a suitable amount of a preservative were added to the resulting solution to form an aqueous phase. Separately, 5 parts of stearic acid, 2 parts of beeswax, 5 parts of cetanol, 13 parts of squalane, and 1 part of lanolin were mixed to form an oil phase. The oil phase was added to the above aqueous phase, and the mixture was pre-emulsified with a homomixer. Then, the pre-emulsified product was emulsified with a Nanomizer (750 kgf/cm²). The emulsion maintained a stable emulsion state though the amount of the fat ingredient used was 8.7 times as much as that of the water-soluble soybean polysaccharide, and it was stable even after six-month storage. The emulsion was applied as a hand cream to the hands. As a result, good refreshed feeling was obtained, and the hand remained moist for a long period of time.

### Example 11

In 70 parts of water, 5 parts of the present invention product prepared in Example 2 was dissolved to obtain an aqueous phase, and 25 parts of O,O-dimethyl-O-(3-methyl-4-nitrophenyl)phosphorothioate as an insecticide ingredient was added thereto. The mixture was pre-emulsified with a homomixer, and then emulsified with a homogenizer (300 kgf/cm²). The emulsion had an average particle diameter of 1.6 µm and maintained a stable emulsion state though the amount of the fat ingredient used was 5 times as much as that of the water-soluble soybean polysaccharide, and it was stable even after six-month storage at 40°C. Further, decomposition of the active ingredient hardly occurred. The emulsion was diluted to concentration of the active ingredient of 250 ppm and applied to completely unfolded leaves of an eggplant, and an insecticidal test was tested using ladybird. As a result, the persistence of the potency was good, and satisfactory results were obtained.

### Example 12

A water-soluble soybean polysaccharide was obtained in the same manner as Example 2 except for using defatted soybeans as a raw material for extraction. When the water-soluble soybean polysaccharide was examined for its emulsifying capability according to the method of Test Example 1, a median diameter equal to or smaller than the desired one was achieved for every system.

### Industrial Applicability

By the production process of the present invention comprising extracting soybeans or a processed soybean product within an acidic region of pH 2.4 to 4.0 with heating to a temperature higher than 100°C, it is possible to obtain a water-soluble soybean polysaccharide having a remarkably improved function as an emulsifier compared with water-soluble soybean polysaccharides obtained by a conventional production process.

## Claims

1. An emulsifier which comprises, as an active ingredient, a water-soluble soybean polysaccharide extracted from soybeans or a processed soybean product at a pH of 2.4 to 4.0 with heating to a temperature of 100°C or higher, wherein the emulsifier has an emulsifying capability sufficient to form a stable emulsion of a composition containing a fat ingredient in an amount 5 times larger than the emulsifier or more in terms of the weight ratio.

2. The emulsifier according to claim 1, wherein the soybeans or processed soybean product are "okara".

3. A process for producing an emulsifier containing as the active ingredient a water-soluble soybean polysaccharide, which comprises extracting soybeans or a processed soybean product at a pH of 2.4 to 4.0 with heating to a temperature of 100°C or higher, wherein the emulsifier has an emulsifying capability sufficient to form a stable emulsion of a composition containing a fat ingredient in an amount 5 times larger than the emulsifier or more in terms of the weight ratio.

4. An emulsified composition using the emulsifier according to claim 1 or claim 2.

## Patentansprüche

1. Emulgator, umfassend als einen aktiven Bestandteil ein wasserlösliches Polysaccharid aus Sojabohnen, das aus Sojabohnen oder einem verarbeiteten Sojabohnenprodukt bei einem pH von 2,4 bis 4,0 unter Erhitzen auf eine Temperatur von 100°C oder höher extrahiert wurde, wobei der Emulgator eine Emulgierfähigkeit aufweist, die ausreicht, um eine stabile Emulsion einer Zusammensetzung, die einen Fettbestandteil in einer 5-fachen oder größeren Menge des Emulgators bezüglich des Gewichtsverhältnisses enthält, zu bilden.

2. Emulgator gemäß Anspruch 1, wobei die Sojabohnen oder das verarbeitete Sojabohnenprodukt "Okara" sind.

3. Verfahren zur Herstellung eines Emulgators, der als aktiven Bestandteil ein wasserlösliches Polysaccharid aus Sojabohnen enthält, wobei das Verfahren die Extraktion von Sojabohnen oder eines verarbeiteten Sojabohnenprodukts bei einem pH von 2,4 bis 4,0 unter Erhitzen auf eine Temperatur von 100°C oder höher umfasst, wobei der Emulgator eine Emulgierfähigkeit aufweist, die ausreicht, um eine stabile Emulsion einer Zusammensetzung, die einen Fettbestandteil in einer 5-fachen oder größeren Menge des Emulgators bezüglich des Gewichtsverhältnisses enthält, zu bilden.

4. Emulgierte Zusammensetzung, die den Emulgator gemäß Anspruch 1 oder Anspruch 2 verwendet.

## Revendications

1. Emulsifiant qui comprend, comme ingrédient actif, un polysaccharide de graines de soja soluble dans l'eau extrait de graines de soja ou un produit de graines de soja traité à un pH de 2,4 à 4,0 avec chauffage à une température de 100°C ou plus, où l'émulsifiant a une capacité d'émulsification suffisante pour former une émulsion stable d'une composition contenant un ingrédient gras en une quantité 5 fois supérieure à l'émulsifiant ou plus en termes du rapport en poids.

2. Emulsifiant selon la revendication 1, où les graines de soja ou le produit de graines de soja traité sont "okara".

3. Procédé pour produire un émulsifiant contenant comme ingrédient actif un polysaccharide de graines de soja soluble dans l'eau, qui comprend l'extraction de graines de soja ou d'un produit de graines de soja traité à un pH de 2,4 à 4,0 avec chauffage à une température de 100°C ou plus, où l'émulsifiant a une capacité d'émulsification suffisante pour former une émulsion stable d'une composition contenant un ingrédient gras en une quantité 5 fois supérieure à l'émulsifiant ou plus en termes du rapport en poids.

4. Composition émulsifiée utilisant l'émulsifiant selon la revendication 1 ou la revendication 2.
